# EUROPEAN PATENT APPLICATION

(11) **EP 1 525 887 A1**
(43) Date of publication of application: **27.04.2005**
(21) Application number: 03745442.8
(22) Date of filing: 28.03.2003
(51) Int. Cl.: A61K 35/78, A61P 35/00, A61P 37/04, A23L 1/30, A23L 1/212

(54) **DRUGS INCLUDING ANTICANCER AGENTS AND IMMUNOPOTENTIATORS AND HEALTH FOODS AND DRINKS**

(30) Priority: 29.03.2002 JP 2002095590
(71) Applicant: SHIROTA, Yasuyuki, Hirosaki-shi, Aomori 036-8252 (JP)
(72) Inventor: SHIROTA, Yasuyuki, Hirosaki-shi, Aomori 036-8252 (JP)
(74) Representative: Tomkinson, Alex
(86) International application number: PCT/JP2003/003944
(87) International publication number: WO 2003/082308

(57) **Abstract**

It has been proven that a fruit of the genus Malus such as apple Malus domestica and crabapple Malus pumila or a fruit of the genus Pyrus, or a substance derived therefrom, when administered, has the actions of enhancing natural immunity of a natural killer cell, a macrophage or the like, activity of lymphocyte, to control cancer-attacked cells, as well as to prevent carcinogenesis of cells not yet attacked by cancer, and when taken in daily life, and further inhibiting the onset of cancer, i.e., having the cancer-preventing effect. It has been further proven that not only a mature fruit of apple, but also an immature fruit, when is used alone or mixed with the mature fruit, has a similar action of inhibiting the onset of cancer.

## Description

### Technical Field

The present invention relates to a medicament such as an anticancer agent or an immune enhancing agent and a health food such as a health food product and a health drink product for inhibiting and preventing cancer or for enhancing immunity, comprising a fruit of the genus Malus such as apple Malus domestica and crabapple Malus pumila or a fruit of the genus Pyrus such as a pear, or a substance derived therefrom as a raw material.

Specifically, the present invention relates to a medicament such as an anticancer agent or an immune enhancing agent, and to a health food such as a health food product and a health drink product for inhibiting and preventing cancer or for enhancing immunity, comprising as a raw material the above-mentioned fruit of the apple genus Malus or the fruit of the genus Pyrus, or a substance derived therefrom, or as processed to juice and the like, or the extract obtained by extracting the above-mentioned fruit of the apple genus Malus or the fruit of the genus Pyrus with water or an organic solvent such as alcohol, or a substance derived from the extract, further a medicament such as an anticancer agent or an immune enhancing agent, and a health food such as a health food product and a health drink product for inhibiting and preventing cancer or for enhancing immunity, comprising a mature fruit and/or an immature fruit of a fruit of the genus Malus such as apple Malus domestica and crabapple Malus pumila or a fruit of the genus Pyrus as a raw material.

### Background Art

Conventionally, it has been known that using such kind of a medicament such as an anticancer agent or an immune enhancing agent, and a health food, an extract of Cordyceps sinensis, when administered, has the effects of enhancing the innate natural immunity of a natural killer cell, a macrophage or the like, and preventing or suppressing cancer by preventing the growth of cancer cells, and when taken in daily life, has the effect of inhibiting the onset of cancer, and that is, the effects of preventing the onset of cancer (for example, see Patent Document 1).

In addition, it has been known that as a health food using an apple juice, is used one which was prepared by mixing the fruit juice and a proantocyanidine-containing substance, powdering the mixed solution to prepare a powdered composition of the fruit juice and preparing a health food containing the powdered composition of the fruit juice as an active ingredient, which has the functions of anti-oxidation activity, anti-mutagenic activity, anti-ulcer activity, deodorization effect for excrement and prevention of a diabetic complication, due to proantocyanidine, in addition to the functions of the ingredients derived from the fruit juice itself (for example, see Patent Document 2).

### [Patent Document 1]

JP-A-1989-228440 (page 1, page 4, Fig. 1)

### [Patent Document 2]

JP-A-2001-275604 (page 1, page 7)

Conventionally, the extract of Cordyceps sinensis has been used as such kind of a medicament and a health food. However, since Cordyceps sinensis is expensive and is not easily available, it has problems in that it cannot be easily administered to anybody.

In addition, there is no description that a conventional health food containing an apple juice has the therapeutic effects of an anticancer agent, an immune enhancing agent or the like.

On the other hand, apple Malus domestica is a fruit tree belonging to the family Rose which is indigenous to the region from West Asia to Middleeast Asia, and which has a long history of having been cultivated for 4000 years.

As apple Malus domestica which is currently cultivated in Japan, there may be mentioned one which was imported in the Meiji dynasty, or one which has been improved in Japan. In Europe, it has been known that the pectin of plant fiber contained in the fruit has the effects of establishing a healthy balance in the intestine as said in the quotation "An Apple a Day Keeps the Doctor Away". Further, an apple has been known to contain a large amount of potassium which has a diuretic action, and fructose or glucose which is a source of energy.

However, medical or pharmacological studies on the pharmacological effects of the apple fruit have been rarely carried out, but the similar studies on a health food containing apple vinegar as a supplementary ingredient, and an immune-reinforcing food using apple Malus domestica have been proposed. However, studies on the anticancer action or the immune enhancing action of the apple fruit have not been conducted, except for the research by Miura et al., School of Medicine at Hirosaki University, which reports the finding that an apple polyphenol has an antitumor effect.

In other words, a specific study related to the pharmacological effects of an apple fruit itself has not been carried out, and the mechanism of the pharmacological effects has not yet been clarified, and further studies on an apple fruit as an anticancer agent or an immune enhancing agent, or further a cancer-preventing agent have not been tried at all.

Currently, the leading cause of death in Japan is cancer, and surgical operation, radioactive treatment, chemical therapy using an anticancer agent, etc. are commonly carried out as a treatment for cancer. However, cancer for which the anticancer agent is effective is limited, and further the side effects from the anticancer agent including death, are becoming socially crucial problems.

On the other hand, humans or animals have inherent immune system which protects the body from pathogenic microorganisms. Since cancer cells are self-derived foreign substances generated in the body, in the case where the immunity is high, the cancer cells are removed by the functions of a natural killer cell, a macrophage or the like.

However, if the immunity is low due to aging or accumulated stress, cancer cells tend to proliferate.

An object of the invention described in claims 1 to 3 and 6 in the present inventions is to enhance the innate natural immunity of a natural killer cell, a macrophage or the like, or the activity of lymphocyte, and to prevent or suppress cancer while inhibiting the growth of cancer cells by using a fruit of the genus Malus such as apple Malus domestica and crabapple Malus pumila or a fruit of the genus Pyrus.

An object of the invention described in claim 4 is to enable suitable administration depending on the situation, in addition to the object of the invention described in claims 1 to 3.

An object of the invention described in claims 5 and 7 is to obtain the higher antitumor effect or an immune enhancing effect, in addition to the object of the invention described in claims 1 to 4 or 6.

### Disclosure of Invention

The present inventors have found that a fruit of the genus Malus such as apple Malus domestica and crabapple Malus pumila or a fruit of the genus Pyrus, or a substance derived therefrom, has an anticancer effect or a cancer-suppressing effect by carrying out various animal tests at first, and then studies on humans, to complete the present inventions.

To achieve the above-described objects, the invention described in claim 1 in the present inventions is a medicament such as an anticancer agent or an immune enhancing agent, which is characterized by comprising a fruit of the genus Malus such as apple Malus domestica and crabapple Malus pumila or a fruit of the genus Pyrus, or a substance derived therefrom as an active ingredient.

The invention described in claim 2 is characterized by the constitution of using an aqueous extract of the fruit of the genus Malus such as apple Malus domestica and crabapple Malus pumila or the fruit of the genus Pyrus, or a substance derived from the aqueous extract, in addition to the constitution of the invention described in claim 1.

The invention described in claim 3 is characterized by the constitution of using an extract of the fruit of the genus Malus such as apple Malus domestica and crabapple Malus pumila or the fruit of the genus Pyrus obtained by extraction with an organic solvent such as alcohol, or a substance derived from the extract, in addition to the constitution of the invention described in claim 1.

The organic solvent such as alcohol herein includes, for example, acetone, ether, ethyl acetate, chloroform, and methylene chloride.

It has been revealed that the actions of invention described in claims 1 to 3 generated from such constitution, consist in that a fruit of the genus Malus such as apple Malus domestica and crabapple Malus pumila or a fruit of the genus Pyrus, or a substance therefrom, or an extract by extracting the fruit with water or an organic solvent such as alcohol, or a substance derived from the extract, when administered, enhances natural immunity of a natural killer cell, a macrophage or the like to control cancer-attacked cells, as well as to prevent carcinogenesis of cells not yet attacked by cancer, and when taken in daily life, and further inhibits the onset of cancer, i.e., has the cancer-preventing effect.

The invention described in claim 4 is characterized by the constitution of administering as a raw material an extract of the fruit of the genus Malus such as apple Malus domestica and crabapple Malus pumila or the fruit of the genus Pyrus or a substance derived from the extract orally, percutaneously, by injection, or via other routes, in addition to the constitution of the invention described in claim 1, 2 or 3.

The action of invention described in claim 4 generated from such added constitution consists in that it enables percutaneous administration, injection administration and administration via other routes, although oral administration is a general administration route, in addition to the action of the invention described in claim 1, 2 or 3.

The invention described in claim 5 is characterized by the constitution of comprising as a raw material either or both of a mature fruit and an immature fruit of the fruits of the genus Malus such as apple Malus domestica and crabapple Malus pumila or the fruit of the genus Pyrus, in addition to the actions of the invention described in claim 1, 2, 3 or 4.

It has been revealed that the action of invention described in claim 5 generated from such added constitution consists in that not only a mature fruit of apple, but also an immature fruit, when is used alone or mixed with the mature fruit, has a similar action of inhibiting the onset of cancer, in addition to the actions of the invention described in claim 1, 2, 3 or 4.

The invention described in claim 6 is a health food for inhibiting and preventing cancer or for enhancing immunity, comprising a fruit of the genus Malus such as apple Malus domestica and crabapple Malus pumila or a fruit of the genus Pyrus, or a substance derived therefrom as a raw material.

It has been proved that the action of invention described in claim 6 generated from such constitution consists in that a fruit of the genus Malus such as apple Malus domestica and crabapple Malus pumila or a fruit of the genus Pyrus, or a substance derived therefrom, when administered, enhances natural immunity of a natural killer cell, a macrophage or the like to control cancer-attacked cells, as well as to prevent carcinogenesis of cells not yet attacked by cancer, and when taken in daily life, further inhibits the onset of cancer, i.e., has the cancer-preventing effect.

The invention described in claim 7 is characterized by the constitution of comprising either or both of a mature fruit and an immature fruit of the fruits of the genus Malus such as apple Malus domestica and crabapple Malus pumila or the fruit of the genus Pyrus as a raw material, in addition to the constitution of the invention described in claim 6.

It has been revealed that the action of invention described in claim 7 generated from such added constitution consists in that not only a mature fruit of an apple, but also an immature fruit, when is used alone or mixed with the mature fruit, has a similar action of inhibiting the onset of cancer, in addition to the action of the invention described in claim 6.

### Brief Description of the Drawings

Fig. 1 is a graph showing a tumor growth curve (mean value) of the mice of each test group after tumor MethA inoculation.
Fig. 2 is a graph showing the phagocytosis ability of a peritoneal macrophage in the mice having no tumor inoculation and in the mice in which tumor has been cured after tumor inoculation.
Fig. 3 is a graph showing the change of the activity of a natural killer cell in a subject orally administered with an apple in a form of a juice every day for 5 weeks.

### Best Mode for Carrying out the Invention

In the following, Example of the present invention will be explained on the basis of Drawings.

This Example shows the case where a fruit juice of apple Malus domestica was orally administered as a fruit of apple the genus Malus.

40 male 4-week-old BALB/c mice (manufactured by CLEA Japan, Inc.) were divided as four test groups (10 mice/group), which were designed as a test group which intakes a fruit juice of apple Malus domestica, a group administered with the extract of the fruiting body of Cordyceps sinensis, a test group administered with a mixture of the extract of the insect body part of Cordyceps sinensis, and a control group which intakes water only, respectively.

100 grams of Cordyceps sinensis were decocted with 1 liter of distilled water for 70 minutes to prepare a stock solution of the extract of Cordyceps sinensis.

The fruit juice of apple Malus domestica was prepared by a juicer with the skin included and the core (including a seed) removed in the water-washed fruit which contains a large amount of polyphenol or quercetin, and was used without heat-treatment or antioxidant treatment.

Both of the extract of Cordyceps sinensis and the fruit juice of apple were frozen and kept at -35° C, thawed out every day, and mixed with edible water.

Tap water sterilized with an autoclave was used as the edible water, which was changed every day. The fruit juice of an apple or the extract of Cordyceps sinensis was mixed in the edible water, and the mice were allowed free access to water. The concentration was all 2% in volume ratio.

The mice were allowed free access to food which was solid feed for mice (manufactured by CLEA Japan, Inc.), and the amount of water taken by the mice was recorded as well as the amount of the food taken.

Using a big cage, 5 mice per cage were caged and raised.

All the mice were identified individually, and each of the body weights was recorded every day.

The cage was kept at a temperature of 25°C and a humidity of 55%, and the illumination was set such that light : dark = 12 hours : 12 hours.

The mice were allowed to have free life from 4-week-old to 9-week-old, and in the middle of ninth week, the mice were inoculated by injecting subcutaneously with 1 × 10⁵ of mouse tumor MethA.

The hairs of the abdominal side face of the inoculation site were shaved before inoculation to carry out precise observation for establishment of the tumor. The short diameter and the long diameter of the tumor were measured with a digital caliper every day, which was used as reference for decision of anticancer effect.

All of the mice which died from the cancer were dissected, investigated for metastasis of tumor to other organs and existence of ascites and kept in a formalin solution, and suitably a histological research was also done.

Life time was prominently prolonged and therefore obvious life-elongation effect was recognized for the mice of the group administered with the fruit juice of apple Malus domestica as compared to the control group administered with water only after inoculation of tumor MethA.

Further, the size of tumor diameter was remarkably reduced as compared to the control group or the other test groups, which indicates definitely the antitumor effect of an apple fruit.

In the following, it will be explained in more detail.

One mouse in the control group administered with water only died first on the 25^{th} day after inoculation of tumor MethA, and then one mouse in the group administered with Cordyceps sinensis died on the 28^{th} day. However, no death was observed after that until 32^{nd} day.

Further, no death was observed in the group administered with the apple fruit juice until 35^{th} day. Days for 50% death was 44 days for the Cordyceps sinensis group and 53 days for the group administered with apple fruit juice as compared to 39 days for the control group, which indicates life-elongation effects of 5 days and 14 days, respectively as compared to the control group.

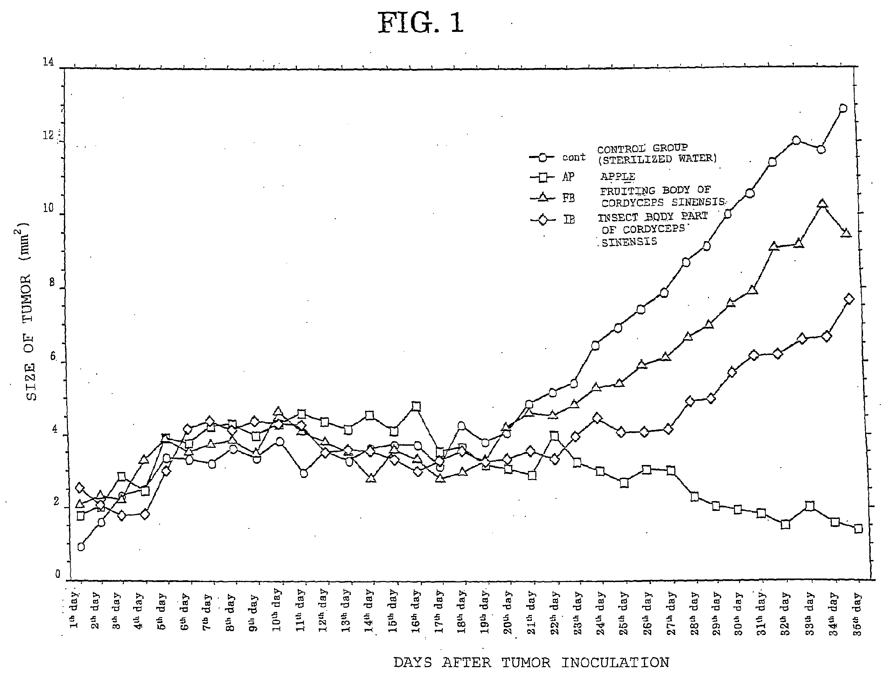

Next, a tumor size change of subjects in each test group until 35 days after inoculation of tumor MethA was shown in Fig. 1.

As the results, as shown in Fig. 1, a tumor growth rate of mice in the group administered with apple fruit juice was suppressed to about 1/4 as compared to the Cordyceps sinensis group, and about 1/10 as compared to the control group administered with water only.

As the results of Fisher's PLSD assay, these values were statistically obviously significant difference.

From the above, it has been proved that the apple fruit juice enhances anticancer effect, and further cancer-suppressing effect.

In addition, the number of mice in which tumor onset was suppressed was 2 in the control group, but 6 in the group administered with apple juice. This indicates that once established tumor is removed by elevated natural immunity of mouse.

Further, it was observed in the group administered with apple juice that the first established tumor was grown to the size of 5.1 mm², and then disappeared.

In addition, mice showing remarkable delaying effect for growth of the tumor were also observed in the group administered with apple juice.

On the other hand, the tumor of the mice in the control group administered with water only continued to grow until it caused death of the subject.

However, it was observed in the group administered with apple juice that the size of the tumor was reduced as well as the growth of the tumor did stop.

This is considered to be due to suppression of tumor growth and necrosis of tumor by oral administration of the apple juice.

From the results of the repeated tests which were newly carried out, it has been proven that the apple fruit juice shows further remarkable antitumor effect.

The survival rate was 30% for the mice in the control group administered with water only, but 80% for the mice in the group administered with apple fruit juice, which was 50% greater than that of the mice in the Cordyceps sinensis group. Further, the life time of the mice where tumor was developed to death was 40 days in the control group, 46.4 days in the Cordyceps sinensis group, but 77 days in the apple group, which indicates about double life-elongation effect.

In addition, the tumor size on the 28^{th} day after tumor inoculation, was 11.0 mm² (square sum of long diameter X short diameter of the tumor) in the control group, 7.5 mm² in the Cordyceps sinensis group, but 1.3 mm² in the apple group, which indicates remarkable suppression of the tumor growth.

These effects are better than those of anticancer hemolytic streptococcus formulation (OK-432; Trademark Picibanil) or an anticancer polysaccharide product (PSK; Trademark Krestin) which has been conventionally used as an immune enhancing agent.

In addition, 20 male 4-week-old BALB/c mice (manufactured by CLEA Japan, Inc.) were divided into two test groups, which were designed as a test group which intakes fruit juice of apple Malus domestica, and a control group which intakes sterilized water only, respectively. The mice which had taken the fruit juice (2% volume ratio) for 3 months every day were inoculated by injecting subcutaneously a carcinogenic substance, azoxymethane.

For further 3 months after that, the apple fruit juice was drunk every day by the mice, which were then dissected to measure the number and the size of the tumor in the esophagus the stomach, and the intestine, and to investigate the influence of the continuous administration of the apple fruit juice on the action of the carcinogenic substance.

As the results, the number of the tumor was 10.6 in the control group, and 6.9 in the apple fruit juice group on the average, so the tumor was suppressed by about 65%, but no statistically significant difference was obtained.

However, the mean value of the surface area which is approximate to oval after measuring the long diameter and the short diameter of tumor, was 162.0 in the control group, and 90.6 in the group administered with apple fruit juice, so the tumor has been proven to be suppressed by about 50%. Further, this difference was statistically significant difference by 3.0% level.

In other words, it has been found that the growth of cancer cells could be suppressed even when inoculated with potent carcinogenic substance such as azoxymethane by continuously drinking the apple fruit juice.

The anticancer and cancer-suppressing effect by oral administration of the apple juice is considered to be from enhancing systemic immunity by the apple fruit or the fruit juice which is absorbed from lymphoid follicle of the digestive tract or epithelial cells of the intestinal mucous membrane. Especially, it has been proven that phagocytosis activity of macrophage is remarkably enhanced.

In the following, phagocytosis activity of macrophage which was carried out by the present inventors, will be explained.

In the above test showing the antitumor effect of the apple juice, 3 mice per test group in which the growth of inoculated MethA was suppressed to cure cancer, were investigated for phagocytosis of a peritoneal macrophage.

The method used was a conventional method, wherein physiological saline was injected into the peritoneal cavity and the recovered macrophage was put into a MEM medium in a Schale, and incubated for 30 minutes in a CO₂ incubator.

Then, it was incubated for 1 hour while giving yeast as food, and May-Grunwald-Giemsa's stain was carried out, meanwhile phagocytosis ability was measured from the number of the yeasts phagocytosized by the macrophage.

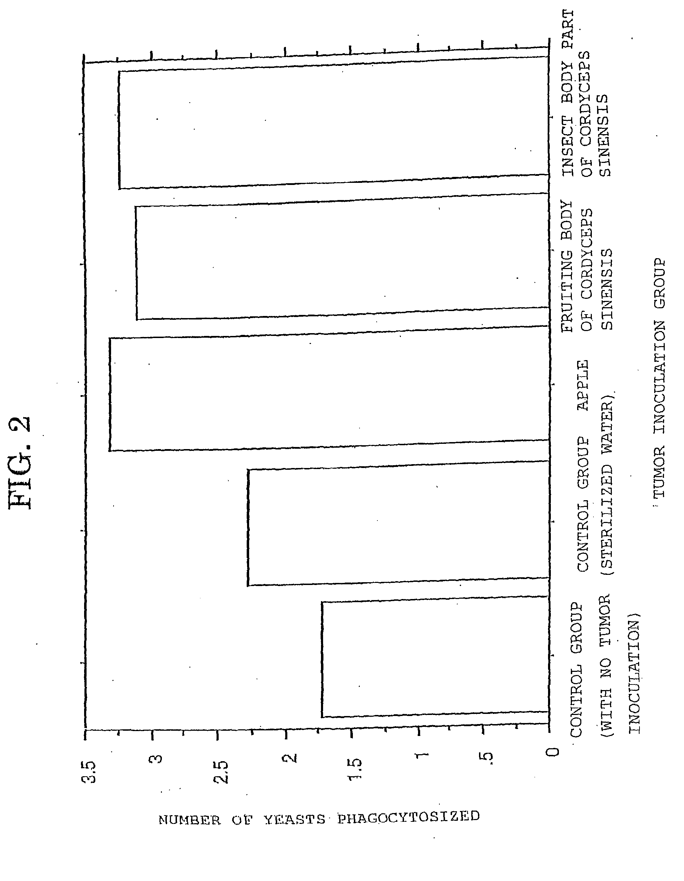

As the results shown in Fig. 2, a macrophage of the mice in the group with tumor inoculation showed the higher phagocytosis as compared to that in the group with no tumor inoculation. Especially, the phagocytosis ability of the macrophage of the mice having taken apple juice was higher than that of mice in the group administered with the extract of Cordyceps sinensis. It was about 1.5 times higher than that of the mice in the group administered with sterilized water only as the control group.

Similar tests were repeatedly carried out for other mice, and as the results, the number of the yeasts phagocytosized by the macrophage of the mice was the average 2.0 in the control group on, 4.0 in the Cordyceps sinensis group, and 4.3 in the apple group which was the highest value. This difference was statistically significant difference. The macrophage in the apple group showed two times of phagocytosis ability as compared to that of the control group.

From these results, it was suggested that by taking apple juice continuously, the phagocytosis ability of macrophage was elevated to attack tumor cells, and therefore, antitumor effect was elevated.

Further, it has been found, as shown below, that frequency or activity of killer T cell or helper T cell in peripheral blood is also elevated from the research for lymphocyte in a peripheral blood using a FACS. Apple fruit juice is considered to enhance the innate natural immunity of a natural killer cell, a macrophage or the like, to inhibit the growth of cancer cells, and to prevent or suppress cancer.

On the other hand, cells taking part in immunity except phagocytes such as a macrophage and a natural killer cell includes the above-described lymphocyte.

The lymphocyte includes a B cell and a T cell, and can identify specifically other cells except for itself. The T cell includes a killer T cell which attacks specifically abnormal cells such as cancer cells, and a helper T cell which helps the action of the killer T cell.

Frequencies of such killer T cell and the helper T cell were investigated in the next.

In the test showing antitumor effect of the apple fruit juice, to the mice in which tumor had been inoculated and cured was continuously administered an apple fruit juice, and then the frequencies of helper T cell and killer T cell in mice were obtained after 5 months.

As the results, frequency of helper T cell of mice was 6% in the control group administered with water only, 30.2% in the Cordyceps sinensis group, and 31.8% in the apple group, which was 5 times greater than the previous ones. However, there was no statistical difference between the Cordyceps sinensis group and the apple group.

Frequency of killer T cell which attacks directly the tumor cells was 1.8% in the control group, 7.7% in the Cordyceps sinensis group, and 10.4% in the apple group, which was the highest in the apple group.

There were statistically significant differences by 3% levels between the apple group and the Cordyceps sinensis group, and by 0.01% of probability between the apple group and the control group. In other words, it is considered that by continuously drinking the apple fruit juice, thereby remove the inoculated tumor MethA, the mice increase frequency of lymphocyte such as a killer T cell which attacks directly cancer cells and a helper T cell which helps it, and thereby elevate phagocytosis ability of a natural killer cell or a macrophage.

The apple fruit juice was divided into the following seven groups to reveal which ingredients contained in the apple fruit juice has the antitumor effect or the immune enhancing effect.
1; Juice from which polyphenol only was removed (pectin, enzyme and unknown substances).
2; Juice obtained by heating said (1) (120°C, 20 minutes) to inactivate the enzyme.
3; Juice from which pectin was removed (polyphenol, enzyme and unknown substances).
4; Juice obtained by heating said (3) (120°C, 20 minutes) to inactivate the enzyme.
5; Juice from which both of polyphenol and pectin were removed (the enzyme and the unknown substances).
6; Juice obtained by heating said (5) (120°C, 20 minutes) to inactivate the enzyme (unknown substances).
7; A control group which is administered with sterilized water only.

Seven test groups were designed as above, and such substances were administered directly to the peritoneal macrophage of the mice, and their influences on phagocytosis ability of the macrophage were investigated.

Further, the test method was a conventional method, wherein 10 µl of a sample was put into 1000 µl (micro-liter) of a MEM medium.

As the results, phagocytosis ability of macrophage was the highest in the test group (3) in which non-heated polyphenol was administered, and it was recognized to have statistically significant difference.

On the other hand, the same 7 kinds of the substances were orally administered to the mice (10 per test group) for 3 months, and then peritoneal phagocytosis ability of macrophage in the mice was investigated, respectively. As the results, the highest value was shown in the group (1) in which non-heated pectin was administered, and it was recognized to have statistically significant difference, as compared to other groups.

In other words, there was discrepancy between the test results when the ingredients of the apple fruit juice were directly administered into macrophage, and the test results when the ingredients were orally administered to the mice and the phagocytosis ability was investigated after the peritoneal macrophage was taken out of the mice. However, it has been proven that both of cases are poorer than the phagocytosis ability of macrophage of mice administered with the apple fruit juice itself, and therefore, administration of the apple fruit juice itself can elevate the antitumor effect or the immune enhancing effect more effectively than separate administration of the ingredients of the apple fruit juice.

In the modern pharmaceutical science or food science, it is a standard method that various substances contained in the apple fruit juice and the like are analyzed, the molecular structures thereof are determined, and these are separately administered to investigate their antitumor effects or immune enhancing effects.

However, it has been proven in this test that such analytic method itself has some problems. In other words, it is considered that various substances act collectively to elevate the antitumor effect or the immune enhancing effect of an apple fruit juice.

It has been proven by the present inventors that an apple fruit juice as it is without separating the apple fruit ingredients into the polyphenol or the pectin, can elevate the antitumor effect or the immune enhancing effect of the fruit.

On the basis of the test results for mice, studies on humans were carried out.

In other words, it was tested if the apple fruit juice as it is, without heat-treatment or antiseptic treatment by vitamin C and the like, has better immune enhancing effect than Cordyceps sinensis or not.

In other words, it was tested that if the apple fruit, with the fruit skin included and the fruit core removed, was made into a juice by a juicer, and taken every day, it would enhance activity of natural killer (NK) cells or not.

A research was planned based on Helsinki Declaration, wherein 37 volunteers (19 male, 18 female) took 2 apples in the form of a juice (Breed; Fuji) every day for 5 weeks. Blood was taken before drinking, 2 weeks after initiation of drinking, 5 weeks after initiation of drinking, and 5 weeks after termination of drinking to investigate NK activity value, blood glucose value, neutral fat value, total cholesterol value, and HDL-C (high cholesterol) value in each of the cases.

As the results, NK value before drinking was 38.8, 2 weeks after initiation of drinking 30.2, 5 weeks after initiation of drinking 46.8, and 5 weeks after termination of drinking 31.7, respectively.

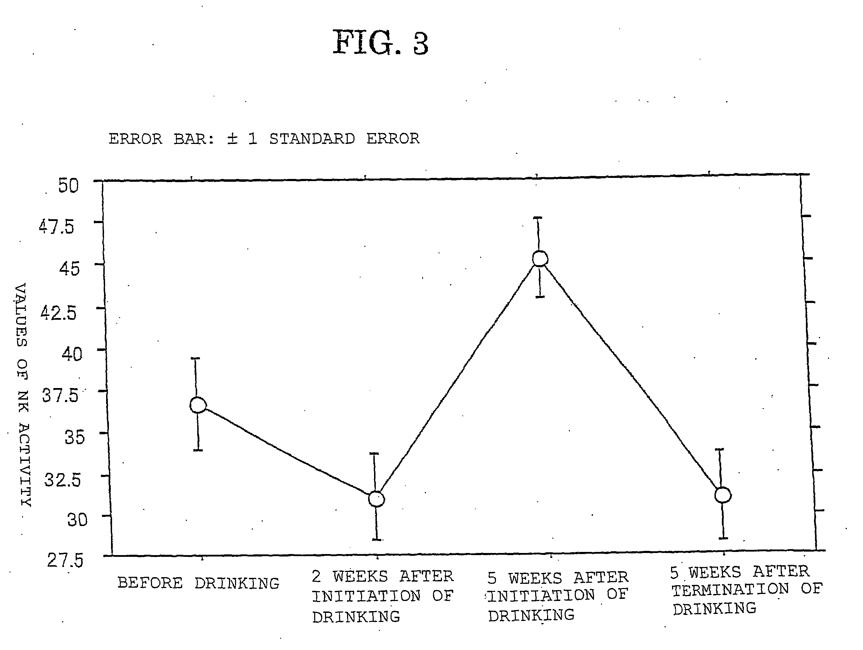

In other words, it has been proven that if the apple fruit juice was continuously drunk for 5 weeks, NK activity value was enhanced in 5 weeks, which was obviously statistically significant as shown in Fig. 3.

Further, this value was greater than the results obtained when a yogurt containing Lactobacillus casei Shirota strain was taken for 3 weeks, which was carried out by Nakao (2000), et al.

In addition, it has been proven that the lower NK activity value of the subject before drinking the apple fruit juice, the higher the NK value was elevated, and abnormally low NK value of the subject was also returned to normal value.

Blood glucose value, neutral fat value, total cholesterol value and HDL-C(high cholesterol) value were compared between before and 5 weeks after taking apple fruit juice, and as the results, it has been proven that blood glucose value was lowered statistically significantly.

By the Examples as above, it has been proven that an apple juice has prominent anticancer and cancer-suppressing effect. Further, it has been proven that the system activate "natural immunity" of a natural killer cell, a macrophage or the like, as well as increase frequency and function of a killer T cell or a helper T cell.

In addition, these effects of the apple fruit have been proven in the research for humans, as well as that for mice.

On the other hand, it has been reported by Tazawa, et al. in Toyama medical and pharmaceutical university that if pectin of an apple fruit and pectin of a mandarin orange are administered to rats and then a carcinogenic substance, azoxymethane is administered, the group administered with the apple pectin showed the higher cancer-suppressing effect.

Further, it has been reported by Eberhardt, et al. in Cornell University, USA that polyphenol of an apple extracted by acetone has much higher anti-oxidation activity, as compared to Vitamin C.

Apple polyphenol simply called above has more than 300 kinds of the substances such as flavonoid, katekin or the like, so it is unclear which substance among these has high anti-oxidation activity.

In the following, specific examples of the apple fruit juice which can be developed as a health food based on the present invention, will be described.

Immature fruits of 1 or 2 months after ripening are thinned out and discarded for harvesting big fruits in the process of so-called "fruit thinning." Such immature fruits have polyphenol about 10 times more per unit weight, as compared to mature fruits.

It was tested that if such immature fruits as a raw material, with the fruit skin included and the fruit core removed, were made into juices by a juicer and taken by mice, they would enhance antitumor effect or not. Further, antitumor effect when juice of such immature fruit is mixed with juice of mature fruit, was also investigated.

In addition, for a fruit of crabapple Malus pumila or the fruit of the genus Pyrus which has more polyphenol per unit weight as compared to apple Malus domestica, the antitumor effect and the immune enhancing effect were investigated in the same manner as in the above-described test. It was made into a juice with the fruit skin included and the fruit core removed by a juicer and taken by mice. Further, antitumor effect was investigated when a fruit of crabapple Malus domestica was made into a juice by a juicer with the fruit skin included, and mixed with a juice of a mature fruit of apple Malus domestica. This was carried out for the purpose of developing a functional food or a medicament which elevates the antitumor effect and the immune enhancing effect by enhancing frequencies of pectin and polyphenol by mixing pectin which is contained in a large amount in the juice of a mature fruit of apple Malus domestica, with polyphenol which is contained in a large amount in a fruit of crabapple Malus pumila or a fruit of the genus Pyrus.

As the results of the mouse test, it has been proven that if a juice of a mature fruit of apple Malus domestica is mixed with a juice of an immature fruit thereof or, with a fruit juice of crabapple Malus domestica or the genus Pyrus, and is administered, it has higher antitumor effect or immune enhancing effect, as compared to a juice of a mature fruit of apple Malus domestica which is administered alone. Further, it has been proven that the mechanism activates "natural immunity" of a natural killer cell, a macrophage or the like, as well as increases frequency and function of a killer T cell or a helper T cell.

In addition, a dog (Siberian husky) born on 27 January, 1989 was found to have tumor having a size of a quail's egg or more at the right lower thigh on 19 March, 2001 (at age 13), which was removed by surgical operation, but was found to spread to the lung (1.5 to 2.0 cm) on 21 May, 2002. The remaining life was diagnosed as 1 to 2 months. The dog which could not even drink water, was orally administered the apple fruit juice made into a juice by a juicer with the fruit skin included and the fruit core removed in early June, 2002. One apple (breed: Fuji) made into a juice by a juicer was continuously administered in the morning and night two times every day. As the result of examination on 5 October 2002, no tumor was found.

Further, a dog (Siberian husky) born on 22 March 1992, was found to have tumor having a size of the end part of a little finger at the very end of the left breast on 21 May, 2002 (at age 10). One apple (breed: Fuji) made to juice by a juicer with the fruit skin included and the fruit core removed was continuously administered in the morning and night two times every day. As the result of examination on 5 October, 2002, no tumor was found.

Apple fruit or juice which has been proven to have anticancer effect in the present invention, has been provided as a food for long time, so it is already obvious that it has no side effect.

As described above, apple fruit or juice, which has high immune enhancing effect, can be used as anticancer agent, as well as a health food product or a health drink product.

As described above, an apple fruit or a juice thereof can prevent cancer when taken in daily life.

As described above, the present invention can save humans from cancer, enhance QOL (Quality Of Life) of cancer patients, thus to contribute greatly to medical care of humans or animal, as well as development of agriculture or apple industry and related process industry and the like.

In the above Examples, juice of the fruit of the genus Malus such as apple Malus domestica or crabapple Malus pumila was orally administered as a fruit of the apple genus Malus. However, the same effects were obtained by the tests even when other substance, a fruit of the genus Malus such as such as Malus sieboldii, or a fruit of the genus Pyrus such as pear or a substance derived from these fruits, or aqueous extract of the fruit of the apple genus Malus or a substance derived from the aqueous extract, extract of the fruit of the apple genus Malus obtained by extraction with an organic solvent such as alcohol, or a substance derived from the extract, was used and administered percutaneously, by injection or via any other routes except an oral route.

In addition, the same effects were obtained by the tests as those of the above Examples even when the above-described fruit such as an apple or a pear was administered in a mixture with for example, a fruit of the genus Prunus such as an apricot, a plum, a peach or a mountain cherry, a fruit of the genus Chaenomeles such as a Flowering quince, a fruit of the genus Cydonia such as a marmelo, a fruit of the genus Diospyros such as a persimmon, a fruit of the genus Morus such as a mulberry, a fruit of the genus Rosa such as a Japanese rose, a fruit of the genus Poncrirus such as a trifoliate orange, or a fruit of the genus Eriobotrya such as a loquat, or even when for example, a fruit of the genus Prunus such as an apricot, a plum, a yellow peach or a mountain cherry, a fruit of the genus Chaenomeles such as a Flowering quince, a fruit of the genus Cydonia such as a marmelo, a fruit of the genus Diospyros such as a persimmon, a fruit of the genus Morus such as a mulberry, a fruit of the genus Rosa such as a Japanese rose, a fruit of the genus Poncrirus such as a trifoliate orange, or a fruit of the genus Eriobotrya such as a loquat, was administered alone.

### INDUSTRIAL APPLICABILITY

As described above, it has been proven that the invention described in claims 1 to 3 or 6 in the present inventions has the effects that a fruit of the genus Malus such as apple Malus domestica and crabapple Malus pumila or a fruit of the genus Pyrus, or a substance derived therefrom, or an extract by extracting the fruit with water or an organic solvent such as alcohol, or a substance derived from the extract, when administered, enhances natural immunity of a natural killer cell, a macrophage or the like to control cancer-attacked cells, as well as to prevent carcinogenesis of cells not yet attacked by cancer, and when taken in daily life, further inhibits the onset of cancer, i.e., has the cancer-preventing effect.

In other words, administration of the fruit of the genus Malus such as apple Malus domestica and crabapple Malus pumila or the fruit of the genus Pyrus, or a substance derived therefrom could enhance the innate natural immunity of a natural killer cell, a macrophage or the like, or the activity of lymphocyte, inhibit onset or growth of cancer cells, and prevent or suppress cancer.

Further, it has been proven that it could suppress or control cancer more effectively than the extract of Cordyceps sinensis which has conventionally been suggested as an immune enhancing agent.

Further, it has been found that by administering a fruit of the genus Malus such as apple Malus domestica and crabapple Malus pumila or a fruit of the genus Pyrus, or a substance derived therefrom to a cancer patient, QOL of the cancer patient is improved, and remarkable life-elongation effect is obtained.

Accordingly, it can be easily administered to anybody since it is not expensive and easily available as compared to the conventional administration of the extract of Cordyceps sinensis.

The invention described in claim 4 enables suitable administration depending on the situation percutaneously, by injection or via other routes, although oral administration is a general administration route, in addition to the effects of the invention described in claims 1 to 3.

The invention described in claims 5 and 7 wherein a mature fruit of apple Malus domestica is mixed with an immature fruit thereof or a fruit of crabapple Malus domestica, has been proven to have the higher antitumor effect or the immune enhancing effect than a mature fruit of apple Malus domestica alone, in addition to the effects of the invention described in claims 1 to 4 or 6.

In addition, if an immature fruit which was conventionally thinned out and discarded is used, it can be harvested and utilized as a juice. In addition, it can be harvested without spreading insecticide or antiseptics thereon since it is harvested before harmful insect or diseases generates.

Therefore, organic fruit juice can be easily prepared with saving cost for agricultural chemicals, and thus it contributes greatly to the industrial development as new raw materials for a health food.

## Claims

1. A medicament such as an anticancer agent or an immune enhancing agent, comprising a fruit of the genus Malus such as apple Malus domestica and crabapple Malus pumila or a fruit of the genus Pyrus, or a substance derived therefrom as an active ingredient.

2. The medicament such as an anticancer agent or an immune enhancing agent as described in claim, 1 comprising an aqueous extract of the fruit of the genus Malus such as apple Malus domestica and crabapple Malus pumila or the fruit of the genus Pyrus, or a substance derived from the aqueous extract.

3. The medicament such as an anticancer agent or an immune enhancing agent as described in claim 1, comprising an extract of the fruit of the genus Malus such as apple Malus domestica and crabapple Malus pumila or the fruit of the genus Pyrus obtained by extraction with an organic solvent such as alcohol, or a substance derived from the extract.

4. The medicament such as an anticancer agent or an immune enhancing agent as described in claim 1, 2 or 3, administered orally, percutaneously, by injection or via other routes, which comprises the extract of the fruit of the genus Malus such as apple Malus domestica and crabapple Malus pumila or the fruit of the genus Pyrus, or a substance derived from the extract as a raw material.

5. The medicament such as an anticancer agent or an immune enhancing agent as described in claim 1, 2, 3 or 4, comprising either or both of a mature fruit and an immature fruit of the fruits of the genus Malus such as apple Malus domestica and crabapple Malus pumila or the fruit of the genus Pyrus as a raw material.

6. A health food for inhibiting and preventing cancer or for enhancing immunity, comprising a fruit of the genus Malus such as apple Malus domestica and crabapple Malus pumila or a fruit of the genus Pyrus, or a substance derived therefrom as a raw material.

7. The health food as described in claim 6, comprising either or both of a mature fruit and an immature fruit of the fruits of the genus Malus such as apple Malus domestica and crabapple Malus pumila or the fruit of the genus Pyrus as a raw material.
